Europäisches Patentamt

European Patent Office

Office européen des brevets

(19)

(11) Publication number: **0 131 932**
**B1**

# (12) EUROPEAN PATENT SPECIFICATION

(45) Date of publication of patent specification: **22.10.86**

(51) Int. Cl.⁴: **C 07 C 19/045, C 07 C 17/38**

(21) Application number: **84108243.1**

(22) Date of filing: **13.07.84**

(54) **Method for distillation of 1,2-dichloroethane.**

(30) Priority: **13.07.83 JP 126246/83**
**15.07.83 JP 127828/83**

(43) Date of publication of application:
**23.01.85 Bulletin 85/04**

(45) Publication of the grant of the patent:
**22.10.86 Bulletin 86/43**

(84) Designated Contracting States:
**DE NL SE**

(56) References cited:
**DE-A-3 112 011**

**HYDROCARBON PROCESSING, vol. 60, March
1981, Houston G.P. QUADRI "Use heat pump
for P-P splitter" pages 147-151**

(73) Proprietor: **TOYO SODA MANUFACTURING
CO., LTD.**
**No. 4560, Ooaza Tonda Shinnanyo-shi
Yamaguchi-ken (JP)**

(72) Inventor: **Yamato, Hiroshi**
**No. 5-8, Betsumei 3-chome
Yokkaichi-shi Mie (JP)**
Inventor: **Kawasaki, Koichi**
**No. 4-10, Betsumei 3-chome
Yokkaichi-shi Mie (JP)**
Inventor: **Takahashi, Isao**
**No. 260-252, Heizushinmachi
Yokkaichi-shi Mie (JP)**
Inventor: **Harada, Haruyuki**
**No. 3527, Ooaza Hazu
Yokkaichi-shi Mie (JP)**

(74) Representative: **Hansen, Bernd, Dr.rer.nat. et al
Hoffmann, Eitle & Partner Patentanwälte
Arabellastrasse 4
D-8000 München 81 (DE)**

## Description

The present invention relates to a method for distillation of 1,2-dichloroethane (hereunder abbreviated as EDC). More particularly, the invention relates to a method for EDC distribution which includes a heat recovery step for making effective use of the thermal energy of an EDC gas being recovered from the top of a high-boiling column in which EDC containing high-boiling substances is distilled.

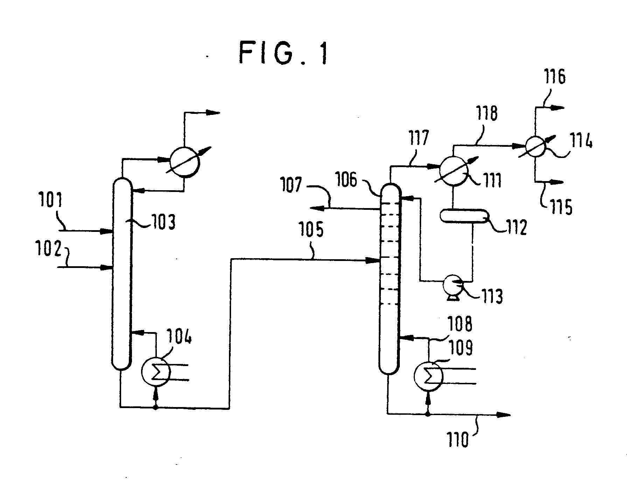

A flowsheet for the conventional method of EDC distillation is shown in Figure 1. Crude EDC containing low-boiling and high-boiling substances is supplied from a dehydrator column and a vinyl chloride column into a low-boiling column 103 through pipes 101 and 102, respectively, for distillation separation of the low-boiling substances. The bottoms from column 103 containing the high-boiling substances are fed to a high-boiling column 106 through a pipe 105. The thermal energy required in the column 103 is supplied from a reboiler 104. The distillate from column 106 is fed to an EDC cracking furnace (not shown) through a pipe 107. The bottoms from 106 are reheated in a reboiler 109 while they are circulating through a pipe 108, and after the reheating, the bottoms ascend through the column 106 and pass through another cycle of distillation.

EDC bottoms containing from 5 to 20 mol% of high boiling substances such as 1,1,2-trichloroethane and 1,1,2,2-tetrachloroethane are fed into an EDC recovery column through a pipe 110. The greater part of the EDC gas discharged from the top of the column 106 and which has a pressure of less than 0.5 kg/cm²G is cooled in a condenser 111 and separated from the uncondensed gas in a gas-liquid separator 112. The condensate is withdrawn from the separator 112 with a pump 113 and refluxed to the top of the column 106.

The EDC gas which has not condensed in 111 and which contains trace amounts of hydrogen chloride and water is cooled in a vent condenser 114, and the condensate is withdrawn through a pipe 115 and the still uncondensed gas is withdrawn through a pipe 116.

The method described above is deficient, e.g., in that a large amount of thermal energy is lost, especially in the condenser 111. However, no effective method is known for recovering and making further use of the thermal energy loss occurring in a condenser connected to the top of an EDC distilling column.

An attempt at heat recovery from a propylene and propane distillation column is known, and this is done by combining a compressive heat pump with the distillation column, as described in *Hydrocarbon Processing*, Feb. 1981, pp. 191—126, and March 1981, pp. 147—151. However, EDC distillation involves entirely different operating conditions with respect to pressure, temperature, etc.

The present inventors have conducted extensive studies on the conditions for heat recovery from EDC gas to be recovered from the high-boiling column, and have finally accomplished the present invention.

The invention provides a method for distillation of EDC containing high-boiling substances in a high-boiling column, comprising distilling the EDC in said high-boiling column at an overhead pressure higher than one atmosphere (absolute), introducing the EDC gas recovered from the top of said column into a compressor, increasing the pressure and temperature of said gas to levels higher than the overhead pressure and temperature by from 0.5 kg/cm² or more and from 7°C or more, respectively, and recovering the heat from said gas.

Figure 1 is a flowsheet for the conventional method of distillation of 1,2-dichloroethane.

Figure 2 is a flowsheet for an embodiment of the present invention.

Figure 3 is a flowsheet for a preferred embodiment of the method of the present invention.

In the Figures, 103 is a low-boiling column, 104 is a reboiler for low-boiling column, 106 is a high-boiling column, 109 is a reboiler for high-boiling column, 111 is a condenser, 202 is a high-boiling column, 203 is a turbo compresor, 205 is a thermosiphon reboiler, 208 is a gas-liquid separator, 210 is a condenser, 212 is a heat exchanger, 303 is a low-boiling column, 305 is a high-boiling column, 307 is a turbo compressor, 309 is a thermosiphon reboiler associated with the low-boiling column, 312 is a gas-liquid separator, 314 is a thermosiphon reboiler associated with the high-boiling column, 317 is a gas-liquid separator, 318 is a reboiler, and 320 and 321 are heat exchangers.

Detailed Description of the Invention

The operating pressure at the top of the high-boiling column is at least one atmosphere (absolute), and preferably between one atmosphere (absolute) and 1.5 kg/cm²G (gauge pressure). If a pressure higher than 1.5 kg/cm²G is selected and the column is operated at elevated temperatures, thermal decomposition of the high-boiling substances in the EDC is promoted, and problems may occur in the EDC refining step.

The pressure and temperature of the EDC gas introduced into the compressor are elevated there by respective degrees of from 0.5 to 7 kg/cm² and from 7 to 80°C. If greater degrees of elevation are selected, not only are the energy and investment cost for the compressor increased, but also thermal decomposition of the compressed EDC gas is accelerated, thus increasing the chances of problems occurring during the EDC refining step. If the degrees of pressure and temperature increase are less than from 0.5 to 7 kg/cm² and from 7 to 80°C, the recovered heat energy finds only limited applications of use and the initial cost of the heat exchanger for heat recovery is increased.

According to the present invention, at least 85% of the heat value of the thermal energy loss that has occurred in the overhead condenser connected to the high-boiling column can be recovered, requiring energy equivalent to only about from 5 to 20% of that thermal loss. The recovery heat source is recycled to reboiler condensers associated, e.g., with the high-boiling and low-boiling columns, and the thermal energy of that source is recovered in such reboiler condensers for subsequent use.

According to a preferred embodiment, the present invention provides a method of distillation for EDC containing high-boiling substances by a sequence including a step of distilling the high-boiling substances and a step of recovering heat from the gas recovered from the top of the high-boiling column, wherein said high-boiling column is operated at an overhead pressure of at least 0.5 kg/cm²G, and the heat recovered is used as a heat source for reboilers associated with at least two columns selected from the high-boiling column, low-boiling column, dehydrator column, EDC recovery column and a vinyl chloride column.

Thus, according to a preferred embodiment of the present invention, the pressure at the top of the high-boiling column is at least 0.5 kg/cm²G, and more preferably the range of from 0.5 to 1.5 kg/cm²G is selected in order to prevent the thermal decomposition of EDC or the high-boiling substances in the EDC.

The EDC gas introduced into the compressor is preferably compressed to an extent of at least 0.5 kg/cm², and more preferably the range of from 0.5 to 2.0 kg/cm² is selected in order to save the energy necessary for pressure boosting and to avoid an increase in the initial cost of the compressor.

According to the preferred embodiment of the present invention, an amount of energy accounting for about 8% of the thermal energy loss occurring in the overhead condenser associated with the high-boiling column is sufficient for recovering the total heat value of that loss and using it as a heat source for reboilers associated with the distillation columns in a vinyl monomer plant including a high-boiling column. By either decreasing the pressure loss in the distillation columns or increasing the heat transfer area of the reboiler condensers, the energy for compression can be made even smaller than 8%.

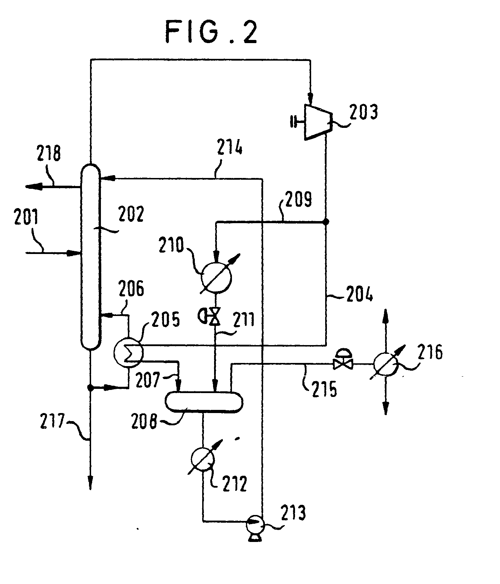

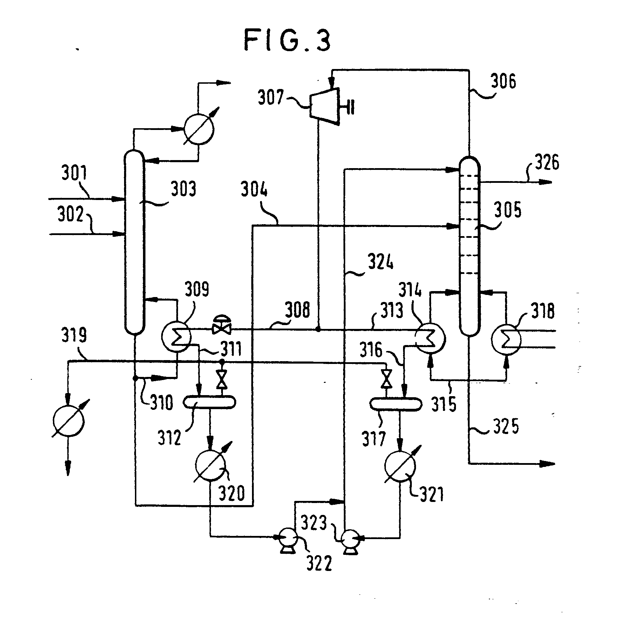

The energy required for operating the compressor in this preferred embodiment of the method of the present invention as illustrated in Figure 3 is about one half the energy required when the heat recovery system shown in the flowsheet of Figure 2 is applied to the high-boiling column. The preferred embodiment has the further advantage that it greatly reduces the initial cost of the compressor.

As a result of operating the high-boiling column at an elevated pressure, the temperature of the distillates, i.e., purified EDC and bottoms EDC, are increased, and the resulting increases in thermal energy are effectively recovered in an EDC cracking furnace and an EDC recovery column to which the purified EDC and bottoms EDC are respectively supplied.

As a further advantage, EDC condenses at a relatively high temperature in the exterior of the shell of a reboiler, and therefore, by recovering the thermal energy involved in the change from the condensation temperature to the temperature at the top of the high-boiling column, a more thermally advantageous process can be realized. For example, by effecting a heat exchange with the bottoms from the dehydrator column and the bottoms from the low-boiling column, the amount of steam necessary for the high-boiling column can be reduced. In another example, the condensed EDC may be heat-exchanged with hot water fed to a steam generator intended for removing the heat of reaction from an oxychlorination zone, and by so doing, more steam can be produced in said generator.

As will be understood from the foregoing description, the present invention provides an industrially very advantageous method for operation of a vinyl chloride monomer plant in that it greatly reduces the required energy consumption and initial cost while ensuring ease in the normal operation of that plant.

The present invention is hereunder described in greater detail by reference to working examples and a comparative example.

Comparative Example

The operation in this comparative example was in accordance with the flowsheet shown in Figure 1.

Crude EDC (45.2 tons/hr) containing 0.7 mol% of low-boiling substances and 0.5 mol% of high-boiling substances was supplied for distribution in a low-boiling column 103 with 85 sieve trays through pipes 101 and 102 while steam (5.9 tons/hr) was supplied into a thermosiphon reboiler 104 having a heat transfer area of 300 m². The bottoms from the column 103 were fed for distillation into a high-boiling column 106 (overhead pressure: 0.05 kg/cm²G) with 51 sieve trays through a pipe 105 while steam (10.3 tons/hr) was fed into a reboiler 109 having a heat transfer area of 600 m².

EDC gas (85°C, 73.4 tons/hr) leaving the top of the column 106 was fed into a condenser 111 through a pipe 117, and 72.8 tons/hr of the EDC gas feed was condensed and refluxed to the top of the column 106 with a pump 113. The heat of condensation of the EDC gas was 5.64 × 10⁶ kcal/hr. The uncondensed EDC gas (0.6 ton/hr) containing trace levels of hydrogen chloride and water was fed into a vent condenser 114 through a pipe 118, and after cooling, the condensate was withdrawn out of the system through a pipe 115, whereas the uncondensed gas was discharged through a pipe 116. The bottoms (95°C, 3.8 tons/hr) from the column 106 containing 5 mol% of the high-boiling substances were supplied into an EDC recovery column (not shown) through a pipe 110.

The refined EDC (85°C) withdrawn as liquid from the top of the column 106 through pipe 107 contained 0.5 mol% of low-boiling substances and 0.01 mol% of high-boiling substances. In the distillation of this comparative example, 10.3 tons of steam was supplied to the column 106 per hour.

### Example 1

The operation in this example was in accordanced with the flowsheet shown in Figure 2.

Crude EDC having the same composition, temperature and flow rate as those of the bottoms extracted from the low-boiling column in Comparative Example was supplied through a pipe 201 to a high-boiling column 202 having the same construction as used in Comparative Example (the stage of distillation at which the crude EDC was supplied was also the same as in Comparative Example), and distillation was performed at an overhead pressure of 0.05 $kg/cm^2G$.

EDc gas (85°C, 73.4 tons/hr) leaving the top of the column 202 was introduced into a turbo compressor 203 wherein the pressure and temperature of said gas were increased to 1.41 $kg/cm_2G$ and 131°C. A portion (71.3 tons/hr) of the thus-treated EDC gas was supplied to the exterior of the shell of a thermosiphon reboiler 205 (heat transfer area: 1,000 $m^2$) through a pipe 204. The supplied EDC gas was subjected to heat exchange at a rate of $5.55 \times 10^6$ kcal/hr with the bottoms (95°C) coming from column 202 through a pipe 206. The resulting condensate was sent to a gas-liquid separator 208 through a pipe 207. In order to maintain the pressure in the column 202 constant, excess thermal energy was removed by cooling the gas in a condenser 210. In this example, 1.5 tons of EDC was supplied hourly to the condenser 210 through pipe 209, condensed there and supplied to the gas-liquid separator 208 through pipe 211.

The EDC gas (0.6 ton/hr) containing trace levels of hydrogen chloride and water leaving uncondensed from the separator 208 was fed to a condenser 216 throgh a pipe 215, and, after cooling, the condensate and uncondensed gas were separately discharged from the condenser.

The liquid EDC (113.5°C) held in the separator 208 was cooled to 85°C in a heat exchanger 212 and subsequently refluxed to the top of the column 202 by a pump 213 through a pipe 214. The bottoms having the same composition and temperature as those of the bottoms obtained in Comparative Example left the column 202 and were supplied to an EDC recovery column through a pipe 217 at a rate of 3.8 tons/hr.

A liquid refined EDC (85°C) was withdrawn from the column 202 through a pipe 218 (the stage of distillation at which said EDC was withdrawn was the same as in column 106 used in Comparative Example); said refined EDC contained 0.5 mol% of low-boiling substances and 0.01 mol% of high boiling substances.

In this example, a power of 910 kWh was required to operate the compressor 203. Column 202 equivalent to column 106 in Comparative Example required no steam as a heat source.

### Example 2

This preferred embodiment, of the process of the present invention is implemented according to the flowsheet shown in Figure 3.

Crude EDC (45.2 tons/hr) having the same composition, temperature and flow rate as those used in Comparative Example was fed into a low-boiling column 303 through pipes 301 and 302. The column 303 had the same construction as that of the column 103 and was operated under the same distillation conditions.

The bottoms from the column 303 were fed into a high-boiling column 305 through a pipe 304. The column 305 had the same construction as that of the column 106 and was operated at an overhead pressure of 1.0 $kg/cm^2G$.

The overhead EDC gas (106°C, 73.4 tons/hr) was fed into a turbo compressor 307 through a pipe 306 and compressed to 2.1 $kg/cm^2G$ and 130°C. The turbo compressor 307 was motor driven at ca 3,600 rpm.

A portion (42.4 tons/hr) of the compressed EDC gas was supplied to the exterior of the shell of a thermosiphon reboiler 309 through a pipe 308. The reboiler 309 was associated with the low-boiling column 303 and had a heat transfer area of 370 $m^2$. In that reboiler, the compressed EDC gas was heat-exchanged with the bottoms (95°C) from the column 303 that circulated through a pipe 310 and the interior of the shell of the reboiler. As a result, the EDC gas condensed at 123°C and the condensate was fed to a gas-liquid separator 312 through a pipe 311. The heat value involved in the heat exchange in the reboiler 309 was $3.14 \times 10^6$ kcal/hr and this was equivalent to 5.9 tons/hr of the steam that was necessary for performing distillation in the low-boiling column 103 in Comparative Example.

The remaining part of the compressed EDC gas (31.0 tons/hr) was supplied to the exterior of the shell of a thermosiphon reboiler 314 through a pipe 313. This reboiler was associated with the high-boiling column 305 and had a heat transfer area of 1,000 $m^2$. In this reboiler, the compressed EDC gas was heat-exchanged with the bottoms (115°C) from the column 305 that circulated through a pipe 315 and the interior of the shell of the reboiler. As a result, the EDC gas condensed at 123°C and the condensate was fed into a gas-liquid separator 317 through a pipe 316. The heat value involved in the heat exchange in the reboiler 314 was $2.29 \times 10^6$ kcal/hr and this was equivalent to 4.4 tons/hr of steam. A reboiler 318 having a heat transfer area of 300 $m^2$ was fed with 6.5 tons of steam per hour, which was equivalent to a heat value of $3.4 \times 10^6$ kcal/hr.

EDC gas containing trace levels of hydrogen chloride and water was discharged from each of the separators 312 and 317 through a pipe 319 at a rate of 0.3 ton/hr.

Liquid EDC (123°C) held in separator 312 (317) was cooled to 106°C in a heat exchanger 320 (321) and refluxed to the top of the high-boiling column 305 through a pipe 324 by a pump 322 (323).

Bottoms (115°C) having the same composition as those obtained in Comparative Example were withdrawn from the high-boiling column 305 and supplied to an EDC recovery column through a pipe 215 at a rate of 3.8 tons per hour.

Purified liquid EDC (40.5 tons/hr, 106°C) obtained from the 48th stage of distillation as counted from the bottom of the column 305 contained 0.5 mol% of low-boiling substances and 0.01 mol% of high-boiling substances and was supplied to an EDC cracking furnace through a pipe 326.

In the operation described above, the power requirement of the compressor 307 was 500 kWh. By operating the high-boiling column 305 at an overhead pressure of 1.0 kg/cm²G which was higher than the value used in Comparative Example 1 and Example 1, the quantity of the heat required for the EDC cracking furnace and EDC recovery column could be reduced by an amount of $3.1 \times 10^5$ kcal/hr, which was equal to 0.6 ton/hr of steam.

While the invention has been described in detail and with reference to specific embodiments thereof, it will be apparent to one skilled in the art that various changes and modifications can be made therein without departing from the spirit and scope thereof.

**Claims**

1. A method for distillation of 1,2-dichloroethane containing high-boiling substances in a high-boiling column, comprising distilling the 1,2-dichloroethane in said high-boiling column at an overhead pressure higher than one atmosphere (absolute), introducing the 1,2-dichloroethane gas recovered from the top of said column into a compressor, increasing the pressure and temperature of said gas to levels higher than the overhead pressure and temperature by from 0.5 kg/cm² or more and from 7°C or more, respectively, and recovering heat from said gas.

2. A method for distillation of 1,2-dichloroethane as in Claim 1, wherein said high-boiling column is operated at an overhead pressure of at least 0.5 kg/cm²G, and the heat recovered is used as a heat source for reboilers associated with at least two columns selected from the high-boiling column, a low-boiling column, a dehydrator column, a 1,2-dichloroethane recovery column and a vinyl chloride column.

3. A method according to Claim 1, wherein the overhead pressure of the high-boiling column is in the range of from 0.5 to 1.5 kg/cm²G.

4. A method according to Claim 2, wherein the overhead pressure of the high boiling column is in the range of from 0.5 to 1.5 kg/cm²G.

5. A method according to Claim 1, wherein the pressure of 1,2-dichloroethane gas introduced into the compressor is elevated to a value from 0.5 to 2.0 kg/cm² higher than the overhead pressure.

6. A method according to Claim 2, wherein the pressure of 1,2-dichloroethane gas introduced into the compressor is elevated to a value from 0.5 to 2.0 kg/cm² higher than the overhead pressure.

**Patentansprüche**

1. Verfahren zur Destillation von 1,2-Dichlorethan, welches hochsiedende Substanzen enthält, in einer Hochsiede-Säule, gekennzeichnet durch Destillieren von 1,2-Dichlorethan in der genannten Hochsiede-Säule bei einem Überkopfdruck von über einer (absoluten) Atmosphäre, Einführen von 1,2-Dichlorethangas, welches im oberen Teil der Säule gewonnen wurde, in einen Kompressor, Erhöhen des Druckes und der Temperatur des genannten Gases auf Werte, die jeweils um 0,5 kg/cm² oder mehr und 7°C oder mehr über dem Überkopfdruck und der Temperatur liegen, und Gewinnung von Wärme aus dem genannten Gas.

2. Verfahren zur Destillation von 1,2-Dichlorethan nach Anspruch 1, dadurch gekennzeichnet, daß die genannte Hochsiede-Säule bei einem Überkopfdruck von mindestens 0,5 kg/cm²G betrieben und die gewonnene Wärme als Wärmequelle für Reboiler verwendet wird, die in Verbindung mit mindestens zwei Säulen stehen, ausgewählt aus der Hochsiede-Säule, einer Niedrigsiede-Säule, einer Dehydrator Säule, einer 1,2-Dichlorethan-Rückgewinnungssäule und einer Vinylchlorid-Säule.

3. Verfahren nach Anspruch 1, dadurch gekennzeichnet, daß der Überkopfdruck der Hochsiede-Säule im Bereich von 0,5 bis 1,5 kg/cm²G liegt.

4. Verfahren nach Anspruch 2, dadurch gekennzeichnet, daß der Überkopfdruck der Hochsiede-Säule im Bereich von 0,5 bis 1,5 kg/cm²G liegt.

5. Verfahren nach Anspruch 1, dadurch gekennzeichnet, daß der Druck des in den Kompressor eingeführten 1,2-Dichlorethangases auf einen Wert erhöht wird, der um 0,5 bis 2,0 kg/cm² höher als der Überkopfdruck liegt.

6. Verfahren nach Anspruch 2, dadurch gekennzeichnet, daß der Druck des in den Kompressor eingeführten 1,2-Dichlorethangases auf einen Wert erhöht wird, der um 0,5 bis 2,0 kg/cm² über dem Überkopfdruck liegt.

**Revendications**

1. Procédé pour la distillation de 1,2-dichloroéthane contenant des substances de haut point d'ébullition dans une colonne à substances de haut point d'ébullition, qui consiste à distiller le 1,2-dichloroéthane dans ladite colonne à substances de haut point d'ébullition à une pression de tête de colonne supérieure à une atmosphère (absolue), à introduire le 1,2-dichloroéthane gazeux récupéré en tête de ladite colonne dans un compreseur, à augmenter la pression et la température dudit gaz à des valeurs supérieures de 0,5 kg/cm² ou plus et de 7°C ou plus, respectivement, à la pression et à la température de tête de colonne et à récupérer la chaleur dudit gaz.

2. Procédé pour la distillation de 1,2-dichloroéthane selon la revendication 1, dans lequel ladite colonne à substances de haut point d'ébullition fonctionne avec une pression manométrique de tête d'au moins 0,5 kg/cm² et la chaleur récupérée est utilisée comme source de chaleur pour les rebouilleurs associés avec au moins deux colonnes choisies parmi la colonne à substances de haut point d'ébullition, une colonne à substances de bas point d'ébullition, une colonne de déshydratation, une colonne de récupération du 1,2-dichloroéthane et une colonne à chlorure de vinyle.

3. Procédé selon la revendication 1, dans lequel la pression manométrique en tête de la colonne à substances de haut point d'ébullition est dans la gamme de 0,5 à 1,5 kg/cm².

4. Procédé selon la revendication 2, dans lequel la pression manométrique en tête de la colonne à substances de haut point d'ébullition est dans la gamme de 0,5 à 1,5 kg/cm².

5. Procédé selon la revendication 1, dans lequel la pression du 1,2-dicloroéthane introduit dans la compresseur est élevée à une valeur supérieure de 0,5 à 2,0 kg/cm² à la pression de tête.

6. Procédé selon la revendication 2, dans lequel la pression du 1,2-dichloroéthane gazeux introduit dans le compresseur est élevée à une valeur supérieure de 0,5 à 2,0 kg/cm² à la pression de tête.

0 131 932

# FIG.1

# FIG.2

# FIG.3